# EUROPEAN PATENT APPLICATION

(11) **EP 4 428 231 A1**
(43) Date of publication of application: **11.09.2024**
(21) Application number: 23792135.8
(22) Date of filing: 18.04.2023
(51) Int. Cl.: C12N 7/00, A61K 39/12, A61P 31/14

(54) **HIGH-TITER JAPANESE ENCEPHALITIS VIRUS GENOTYPE 5 AND USE THEREOF**

(30) Priority: 20.04.2022 KR 20220048696; 08.09.2022 KR 20220114175
(71) Applicant: Korea National Institute of Health, Cheongju-si, Chungcheongbuk-do 28159 (KR)
(72) Inventor: SHIM, Sang Mu, Cheongju-si, Chungcheongbuk-do 28159 (KR); LEE, Jung Min, Cheongju-si, Chungcheongbuk-do 28159 (KR); LEE, Joo Yeon, Cheongju-si, Chungcheongbuk-do 28159 (KR); RYOU, Jungsang, Cheongju-si, Chungcheongbuk-do 28159 (KR)
(74) Representative: Gassner, Birgitta
(86) International application number: PCT/KR2023/005204
(87) International publication number: WO 2023/204559

(57) **Abstract**

The present invention relates to high titer Japanese encephalitis virus genotype 5 and uses thereof, and specifically relates to a high titer virus produced using subculture and mouse cerebral inoculation methods and a vaccine composition comprising the same.

## Description

### BACKGROUND

### Technical Field

The present invention relates to high titer Japanese encephalitis virus genotype 5 and uses thereof.

### Background Art

Japanese encephalitis is an infectious disease transmitted by mosquitoes and is a fatal zoonotic disease that causes encephalitis accompanied by high fever and coma in humans, ultimately leading to death. Japanese encephalitis is known to be spread by culex pipiens (*Culex. tritaeniorhynchus*) in Korea and Japan, but *Culex. annulus, Culex. pipiens*, *Culex. gelidus* and *Anopheles spp.* mosquitoes are also known to transmit the Japanese encephalitis virus in Southeast Asia, including China and India. Unlike adults who show inapparent infection, when children under the age of 14 or the elderly are infected with Japanese encephalitis, it causes high fever, headache, paresthesia, impaired consciousness, coma, and death, and after-effects such as language impairment and impaired judgment appear during the recovery period. Livestock including pigs, horses, cattle, sheep, and goats, as well as wild birds such as pigeons and herons, and reptiles are susceptible to the disease, in particular, pigs are known to be amplifier hosts for the Japanese encephalitis virus, making them an important immunization animal for public health. When the Japanese encephalitis virus is infected by mosquitoes in adult and finishing pigs, it causes viremia but shows inapparent clinical symptoms. However, when a pregnant sow is infected with Japanese encephalitis, it causes a reproductive disorder that causes premature birth, mummified fetuses, and weak piglets with neurological symptoms and similar birth symptoms. In boars, it invades the genital tract and inhibits sperm formation, reducing the sperm count and increasing the number of malformed sperm, thereby lowering the conception rate.

In Korea, Japanese encephalitis is a zoonosis transmitted by mosquitoes that has been occurring continuously since the 1950s, and has mainly occurred in Southeast Asia, including China, Japan, and Korea. However, it has been reported in countries other than Asia, such as Australia, Africa, and Russia, so it is recognized as a new infectious disease that is reemerging, and in the case of humans, approximately 67,900 cases are reported each year in Asia alone, of which approximately 5,000 people die. In particular, recent global warming and climate change, along with the development of agricultural waterways and the specialization and scale of agriculture, have changed the ecological environment of mosquitoes, which are disease vectors, which can have a significant impact on the spread of Japanese encephalitis, making it more problematic. Additionally, when pigs are infected with the Japanese encephalitis virus, they show viremia for 2 to 3 days, and when mosquitoes suck blood during this period, a large amount of the virus is transferred to the mosquitoes. The Japanese encephalitis virus transmitted from pigs can infect other animals or humans through the mosquito's salivary glands after about 10 days in the mosquito's body. Therefore, the prevention of Japanese encephalitis and the establishment of immunity in pigs, which are amplification hosts, are very important in the management of Japanese encephalitis in humans.

The Japanese encephalitis virus currently prevalent in Korea is genotype 1, and the vaccine is administered for genotype 3. In 2015, genotype 5 (isolate name: K15P38) was first isolated from a patient infected with Japanese encephalitis virus in Korea, raising concerns about substitution of existing prevalent viruses. Therefore, it is necessary to prepare for new genotype (genotype 5) substitution, and the present invention sought to develop a vaccine candidate using genotype 5 virus isolated from a patient.

Korean Patent No. 1642705 relates to a vaccine composition for preventing Japanese encephalitis in swine comprising an attenuated genotype 1 Japanese encephalitis virus, and discloses an attenuated genotype 1 Japanese encephalitis virus KV1899-120P (accession number KCTC18347P) and a vaccine composition comprising the same. Korean Patent No. 1484605 discloses an inactivated vaccine composition for Japanese Encephalitis comprising Japanese encephalitis virus KV1899 strain antigen inactivated by binary ethyleneimine (BEI) and swine GM-CSF recombinant protein.

However, the high titer Japanese encephalitis virus genotype 5 of the present invention and uses thereof have not been disclosed.

### DETAILED DESCRIPTION OF THE INVENTION

### Summary

In order to solve the problems, the purpose of the present invention is to secure a high titer with improved proliferation rate in cells using a virus obtained from domestic Japanese encephalitis patients and develop it as a vaccine candidate, in order to prepare for the new Japanese encephalitis virus genotype 5 epidemic.

### Technical Solution

In order to achieve the object of the present invention, the present invention provides high titer Japanese encephalitis genotype 5 K15P38-KNIH.

The Japanese encephalitis virus of the present invention, named K15P38-KNIH, is derived from the Japanese encephalitis virus that has recently become prevalent in Korea, and was obtained by subculture the K15P38 virus strain in Vero cells to obtain the K15P38-V25 virus strain, which was finally produced by inoculating the mouse cerebrum. The results of genome sequence analysis are as shown in SEQ ID NO: 1.

The Japanese encephalitis virus may have accession number KCTC15027BP.

The high titer may be 1×10⁸ pfu/mL or more.

In another example of the invention, the present invention provides a method for producing high titer Japanese encephalitis virus genotype 5 K15P38-KNIH, comprising:
The step of inoculating the mouse cerebrum with the isolated Japanese encephalitis virus; and
The step of obtaining Japanese encephalitis virus from mouse cerebrum;
Wherein the isolated Japanese encephalitis virus is Japanese encephalitis virus genotype 5, and may further comprise a step of subculture in Vero cells, wherein the inoculation may be done twice or more.

In another example of the present invention, the present invention provides a vaccine composition comprising the Japanese encephalitis virus.

Wherein the vaccine composition may further comprise a pharmaceutically acceptable carrier or excipient in addition to the virus. Suitable carriers for vaccines are known to those skilled in the art and include, but are not limited to, proteins, sugars, etc. Wherein the carriers may be an aqueous or non-aqueous solution, suspension or emulsion. Examples of the non-aqueous carriers include propylene glycol, polyethylene glycol, edible oils such as olive oil, and injectable organic esters such as ethyl oleate. The aqueous carriers comprise water, alcohol/aqueous solutions, emulsions or suspensions comprising saline and buffered media. Parenteral carriers comprise sodium chloride solution, Ringer's dextrose, dextrose and sodium chloride, lactated Ringer's or fixed oils.

Carriers for intravenous injection comprise electrolyte supplements, liquid and nutritional supplements, such as those based on Ringer's dextrose. Preservatives and other additives may comprise, for example, antimicrobial agents, antioxidants, chelating agents, inert gases, etc. Preferred preservatives include formalin, thimerosal, neomycin, polymyxin B and amphotericin B.

Also, the vaccine composition may additionally comprise an adjuvant (immune composition, adjuvant) in the form of a diluent.

Wherein the adjuvant refers to a compound or mixture that enhances the immune response and/or accelerates the rate of absorption after inoculation and includes an optional absorption-promoting agent. Acceptable adjuvants comprise Freund's complete adjuvant, Freund's incomplete adjuvant, saponins, mineral gels such as aluminum hydroxide, surfactants such as lysolecithin, pluronic polyols, polyanions, peptides, oil or hydrocarbon emulsions, keyhole limpets, hemocyanin, dinitrophenol, etc., but is not limited to. A preferred adjuvant is MONTANIDE IMS1313 adjuvant from SEPPIC.

The dosage of the vaccine composition for the purpose of the present invention is not limited as it can be adjusted depending on the condition of the administration subject, the administration route and the form of administration, and those skilled in the art can use within the various ranges depending on the symptoms, but typically, in the present invention, it is considered possible to administer 0.01 to 1 ml per 1 kg of body weight (10^{5.0} TCID₅₀ / ml or more), preferably 0.01 to 0.03 ml per 1 kg of body weight, continuously or intermittently per day as an experimental effective amount.

The vaccine composition of the present invention can be administered through oral, intramuscular, subcutaneous, peritoneal, intravenous, dermal, ocular, intracerebral, etc. administration routes, but is not limited thereto, and is preferably administered through the intramuscular route.

In another example of the present invention, the present invention provides a method of vaccination against Japanese encephalitis comprising the step of administering the vaccine composition to a non-human animal.

### EFFECTS OF THE INVENTION

The present invention secures high-titer viruses through repeated inoculation of human isolates into animal cells and mouse brains in order to improve the proliferation rate of vaccine strains, which is a prerequisite for industrial use for vaccine development. Although the growth-improving strain has some genetic mutations, it maintains immunogenicity compared to the original virus, so it will be highly useful as a vaccine candidate.

### BRIEF DESCRIPTION OF THE DRAWINGS

Fig. 1 shows a comparison of the proliferation rates of Japanese encephalitis virus K15P38-V25 and K15P38-KNIH.
Fig. 2 shows a comparison of the immunogenicity of Japanese encephalitis virus K15P38-V25 and K15P38-KNIH viruses. Each virus inoculation serum IgG ELISA. (A) Original Japanese encephalitis virus attachment, (B) K15P38-KNIH virus attachment, control (Ctrl) is non-immune mouse serum.
Fig. 3 shows the analysis results of neutralizing antibody activity of Japanese encephalitis virus K15P38-KNIH inoculated serum.
Fig. 4 shows the results confirming the increase in immunoglobulin antibody titer (total IgG) when inoculated twice with the same antigen in groups vaccinated with each antigen (8 µg/dose) for Japanese encephalitis virus genotypes 3 and 5.

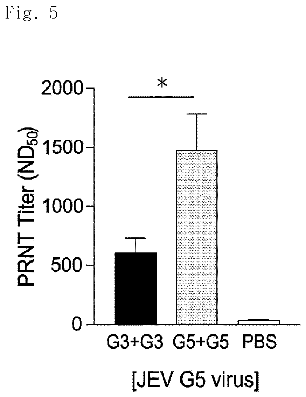

Fig. 5 shows the results confirming neutralizing antibody titer (ND₅₀, Karber formula) using mouse serum induced by genotype 5 immune antigen.

### DETAILED DESCRIPTION

Hereinafter, the present invention will be described in more detail through examples. However, the following examples are for illustrating the present invention and the scope of the present invention is not limited to the following examples.

### <Example 1> Method for manufacturing Japanese encephalitis virus strain

### A. Subculture strain (K15P38-V25)

- Repeated subculture experiments were performed in Vero cells to produce a high-titer virus using K15P38, a genotype 5 Japanese encephalitis virus strain. The K15P38 virus strain is an original virus isolated from a patient. Vero (derived from monkey kidney epithelial cells) cells were prepared by inoculating 5×10⁶ cells/75 flasks. After 24 hours, the cells were washed twice using DPBS, then 200 µl was inoculated into 3 ml serum-free MEM medium and cultured in 5% CO₂ for 1 hour. The total culture medium was added to 6 ml of 2% serum MEM and cultured in 5% CO₂ for 48 hours.

The process was repeated subculture 25 times to produce K15P38-V25.

### B. Mouse cerebral inoculation strain (K15P38-KNIH)

- This experiment was conducted to produce high-titer virus through repeated administration of K15P38-V25 virus to the cerebrum of BALB/c mice. After preparing 5 animals (females) per set, they were anesthetized by intramuscular injection using an anesthetic (zoletyl: 50 µl/kg, lumpun: 10 mg/kg mixed). After making a 1 cm incision in the skin of the head, the head of the mouse was fixed in a deep brain fixation device (Stereotaxic) for small animals. After checking the bregma, the skull on the right side of the hippocampus area was drilled and 5 µl of K15P38-V25 was administered over 2 minutes (2.5 µl/min). The skin was sutured and recovery was induced. Symptom monitoring was confirmed from K15P38-V25 administration (day 0) to the 5th to 6th day. When abnormal symptoms (hair loss, gait abnormality, extension) occurred, mouse brain tissue was removed. 1 ml of PBS was added to the brain tissue, the tissue was disrupted, centrifuged (12,000 rpm, 20 minutes), and the separated supernatant was inoculated again into the mouse. The process was repeated.

### <Example 2> Confirmation of virus titer by cell subculture and repeated inoculation of mouse cerebrum

### A. Confirmation of virus titer through plaque assay method

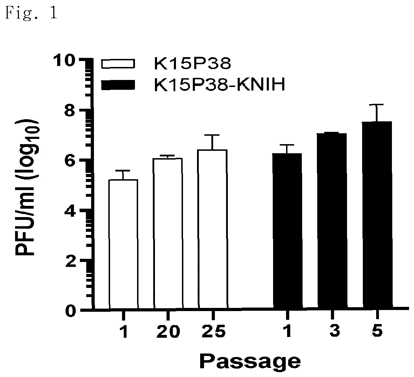

- The titers of viruses obtained by subculture using Vero cells and repeated cerebral inoculation after subculture of Vero cells were compared. As a result of repeated subculture of the Japanese encephalitis genotype 5 isolate only in cells (Fig. 1, black block), the maximum titer was 6.5 × 10⁶ pfu/ml as a result of cell subculture repeated 1, 20, and 25 times, but after repeated mouse brain inoculation, the proliferation rate was improved by subculture to vero cells 1, 3, and 5 times (Fig. 1, white block), and the titer was confirmed to be 1.15 × 10⁸ pfu/ml.
- When the clinical sample isolate (K15P38) virus was subcultured in Vero cells (named K15P38-V25), it was difficult to improve the proliferation rate, but afterwards, it was confirmed that the virus that had been inoculated into the mouse brain, (name K15P38-KNIH, accession number KCTC 15027BP) had an improved proliferation rate and a high titer value. Higher virus titers could be confirmed even with a short subculture in cells after repeated passages in the mouse cerebrum than titers subcultured in cells (Fig. 1).

### <Example 3> Confirmation of genetic mutations in K15P38-V25 and K15P38-KNIH viruses

### A. Japanese encephalitis virus full-length gene mutation analysis

- In order to subculture Vero cells (passages 1 and 25) and to analyze the base sequence of Japanese encephalitis virus after repeated cerebral inoculations to secure high titer, full-length gene mutations were analyzed using Blast 2 sequence. As a result of the analysis, 98% (10925/10945) of Japanese encephalitis virus DNA identities were confirmed to have some genetic mutations (Table 1). The sequence of the final secured virus strain (K15P38-KNIH) is shown in SEQ ID NO: 1 below.

**[Table 1]**

| **Virus type** | **Sequence No.** | | | | | | | | | |
|---|---|---|---|---|---|---|---|---|---|---|
| | **231** | **238** | **283** | **289** | **290** | **539** | **757** | **1338** | **1628** | **1817** |
| patient isolate Origin (K15P38) | G | G | G | G | G | C | C | A | C | A |
| cell passage (K15P38-V25) | G | G | G | G | G | C | C | A | T | A |
| mouse (K15P38-KNIH) | T | T | T | A | A | G | T | C | C | G |

| | **3552** | **3583** | **4067** | **4998** | **5004** | **5005** | **5010** | **5013** | **8361** | **10097** |
|---|---|---|---|---|---|---|---|---|---|---|
| patient isolate Origin (K15P38) | T | - | - | C | C | A | C | T | T | G |
| cell passage (K15P38-V25) | T | - | - | T | T | T | T | A | T | A |
| mouse (K15P38-KNIH) | G | A | A | C | C | A | C | T | C | G |

[SEQ ID NO: 1] K15P38-KNIH full genome sequence

TAGAAGTTTATCTGTGTGAACTTCTTGGCTTAGTATTGTTGAGAGGAATCGAGAGATTAGTGCAGTTTAAACAGT
TTTTTAGAACGGAAGAAAACCATGA 100
CTAAAAAACCAGGAGGGCCCGGTAAAAACCGGGCTATCAATATGCTGAAACGCGGCATACCCCGCGTATCCCCAC
TTGTGGGGGTGAAGAGGGTAATTAT 200
GAACTTGCTCGACGGCAGAGGGCCAATACGATTCGTTTTGGCTCTCTTGGCGTTTTTCAATTTCACATCACTAGC
CCCGACCAAGGCACTCGTTAGCCGA 300

TGGAAGGCAGTATAGAAAAACGTTGCAATGAAACATCTCACCAGTTTCAAAAAGGAACTGGGAACGCTCATCAAC
GCTGTAAATAAGAGGGGCAAAAAAC 400
AAAACAAAAGAGGAGGAAGTAATGGGACAGTCATCTGGATAATGGGCTTGGCAGTTGTGGTTGCCAGTGTGAGTG
CAATCAAGCTGTCAAATTTTCAAGG 500
CAAGGTGCTGATGACAATCAACAACACCGATGTGGCTGATGTGATCACCATTCCCACCTCAAAAGGGAGTAATAG
ATGTTGGGTCCGAGCTATAGATGTG 600
GGACACATGTGCGAGGACACAATCACTTATGAATGCCCAAAGCTTGACGCTGGCAACGACCCGGAGGACATTGAC
TGCTGGTGCGACAAACAAGCCGTGT 700
ATGTCCAGTATGGACGTTGCACTAGGACCAGGCACTCCAGGAGAAGTAGAAGATCTGTGTCAGTGCAAACCCACG
GAGAAAGTTCTTTAGTGAACAAAAA 800
AGAAGCCTGGATGGATTCGACGAAAGCCACTCGGTATCTCATGAAAACAGAGAATTGGATCATACGGAATCCAGG
CTATGCTCTTGTGGCAGTGGCACTT 900
GGATGGATGCTTGGTAGCAACAACGGCCAGCGTGTGGTATTCACAATTCTCTTGCTGTTGGTCGCACCCGCATAT
AGCTTCAACTGCTTAGGTATGGGAA 1000
ATCGTGACTTCATCGAAGGAGTCAGTGGAGCGACGTGGGTGGACCTGGTGCTGGAAGGAGACAGTTGCCTCACCA
TCATGGCGAACGACAAACCAACATT 1100
GGACGTGCGCATGATAAACATCGAGGCCACGGAACTGGCTGAGGTGCGAACCTACTGCTACCACGCCACAGTGGC
TGACATCTCAACAGTGGCAAGATGC 1200
CCCACGACTGGAGAAGCCCATAACACGAAGCGAGCTGACAGCAGCTATGTCTGCAAGCAAGGCTACACTGATCGT
GGATGGGGAAACGGATGTGGGTTGT 1300
TTGGGAAAGGCAGCATTGACACATGCGCTAAATTTGTTTGCAGCCACAAGGCCATTGGAAAGATAATCCAGCCAG
AAAACATCAAATATGAAGTTGGAGT 1400
GTTTGTTCATGGAACCACAACAGCAGAGAACCACGGAAATTACACAGCCCAGATTGGGGCTTCCCAGGCTGCCAA
GTTCACCATCACGCCCAATGCTCCT 1500
TCCATCACTCTGAAGCTTGGAGATTACGGAGAAGTCACGATGGATTGCGAGCCGCGTAGTGGATTTAATACTGAG
GCATTTTACGTGCTGACCGTAGGGT 1600
CTAAGTCGTTCCTAGTCCACCGCGAATGGTTCAATGATCTTGCGCTTCCGTGGCTATCTCCATCTAGCACAAATT
GGAGAAATAGAGAGATCCTGATGGA 1700
GTTCGAAGAGGCTCACGCGACAAAACAGTCTGTCGTCGCGCTTGGATCACAAGAGGGAGCCTTGCATCAGGCATT
GGCTGGTGCCATAGTGGTGGAGTAC 1800

TCTAGCTCAGTGAAGTTGACATCTGGTCACCTCAAATGCAGACTAAGAATGGAGAAGTTGGCGTTGAAAGGGACC
ACCTATGGTATGTGCACAGAGAAGT 1900
TCTCTTTTTCCAAGAATCCAGCTGACACTGGTCATGGTACGGTTGTCATAGAATTGCAGTACACCGGCACTGACG
GACCTTGCAAGATACCCATTTCTTC 2000
GGTGGCCAGTCTGAATGATTTAACTCCAGTTGGTAGATTGGTGACAGTCAATCCTTTTGTTGCCACATCCACCGC
CAATTCGAAGGTTTTGGTAGAATTG 2100
GAACCACCATTTGGAGATTCATTCATTGTTGTCGGAAGAGGAGATAAGCAGATCAATCACCATTGGCACAAGGCT
GGCAGTTCACTGGGAAAGGCTTTCA 2200
CGACTACTTTGAAAGGAGCTCAGAGGCTGGCAGCCCTTGGTGACACCGCTTGGGACTTTGGATCCATTGGAGGGG
TTTTCAATTCCATTGGTAAGGCCGT 2300
GCACCAAGTGTTTGGAGGAGCTTTCAGAACCCTTTTTGGTGGCATGTCCTGGATAACACAAGGATTGATGGGAGC
GCTGCTGCTGTGGATGGGTATCAAC 2400
GCACGAGATCGGTCGATTGCACTAGCTTTTCTTGCTACAGGAGGTGTGCTCTTGTTTCTGGCCACCAATGTCCAC
GCCGATACCGGCTGCGCCATCGATA 2500
TAACCAGAAAAGAAATGAGGTGTGGTAGTGGCATATTTGTGCACAATGACGTGGAGGCTTGGGTTGATAGATACA
AGTATCTACCTGAGACTCCCAAGTC 2600
TTTAGCCAAAATAGTCCACAAAGCACACAAGGAAGGCATTTGTGGAGTGAGATCAGTCACCAGACTGGAACACCA
AATGTGGGAGGCCGTCAGGGACGAG 2700
TTAAATGTCTTGTTGAAGGAGAATGCGGTAGATCTTAGTGTGGTGGTGGACAAACCATCAGGAAGATACCGACCA
GCTCCATTGCGGTTGGCCATGACTC 2800
AGGAAAAGTTTGAGATGGGTTGGAAAGCATGGGGGAAGAGCATTCTCTTTGCGCCGGAACTGGCCAATTCCACGT
TTGTGATCGACGGACCTGAAACCAA 2900
AGAGTGTCCAGATGAGCGTAGAGCATGGAACAGCATGCAGATTGAGGATTTTGGGTTTGGCATTACGTCGACTCG
AGTGTGGTTGAAGATCAGGGAGGAG 3000
CGCACGAATGAATGTGATGGAGCCATCATCGGCACGGCTGTTAAAGGGAACATGGCAGTGCACAGTGACTTGTCA
TACTGGATTGAAAGCCATCTCAACG 3100
ACACCTGGAAGCTCGAGAGAGCTGTGTTTGGAGAGATAAAGTCTTGCACTTGGCCAGAAACACACACGCTCTGGG
GAGATGGTGTTGAGGAAAGTGAGTT 3200
GATAATTCCACACACACTCGCTGGACCCAAAAGCAAGCATAACAGAAGAGAGGGTTACAAGACACAGAATCAGGG
ACCATGGGACGAGAGTGAGATCACT 3300

CTTGATTTTGACTACTGTCCAGGGACCACAGTCACCATTGCTGAGGGATGTGGGAAAAGGGGGCCCTCAATCAGG
ACCACCACTGATAGTGGAAAATTAA 3400
TCACTGATTGGTGTTGCAGGAGCTGCACTTTGCCGCCACTGAGGTTCAGGACAGCCAGTGGGTGCTGGTATGGAA
TGGAAATACGGCCCATGAAgCATGA 3500
TGAATCCACGCTCGTGAAATCACAAGTCAACGCGTTTAATGGGGAGATGATTGATCCTTTTCAGTTGGGCCTTCT
GGTGATGTTTCTGGCCACCCAGGAG 3600
GTCCTTCGCAAGAAGGTGGACGGCCAGACTGACGATTCCTGCGGTTTTGGGGGCCCTACTTGTTCTGATGCTTGG
GGGCATCACCTACACTGATCTAGTG 3700
AGATATGTGGTGTTGGTGGCTGCTGCTTTCGCTGAAGCTAACAATGGAGGCGATGTAGTTCATTTGGCCCTGATT
GCCGTATTTAAAATCCAACCGGCAT 3800
TCCTAGTCATGAGCATAGCAAGAACAAATTGGACAAACCAGGAAAACATTGCCCTAGTGCTAGGAGCTGCTTTCT
TTCAGATGGCCTCAACAGACCTGGA 3900
GTTTGGTATCCATGGGCTGCTAAATGCAGCGGCGACGGCCTGGATGGTAGTGCGGGCGATCACGTTTCCCACGAC
CTCCACCATCACAATGCCCATACTA 4000
GCTTTGCTGGCGCCAGGAATGAGAGCTCTCCATCTTGACACCTACAGAATCTTTTTGCTTATCATCGGGGTCTGT
GCTCTGCTGCATGAAAGGAGGAAAa 4100
aCCATGGCGAAGAAGAAAGGTGCTGTCCTTTTGGGTCTAGCCCTAAGTTCCACTGGGTGGTTTTCACCAGCCATT
ATGGCAGCTGGGCTCATGGCGTGCA 4200
ATCCAAACAAAAAGAGAGGATGGCCAGCGACAGAATTTCTGTCTGCGATTGGGTTGATGTTTGCTATTGTTGGGG
GCCTGGCCGAGTTGGACATCGACTC 4300
TATGGCGATACCTTTCATGTTAGCTGGGCTTATGGCAGTGTCATATGTGGTATCAGGAAAAGCAACGGACATGTG
GCTAGAACGTGCGGCCGACATTAGT 4400
TGGGAAGTGGATGCAGCGATCACAGGCAGCAGCCGAAGGTTGGACGTCAAACTAGATGATGATGGAGATTTCCAC
CTTATTGATGATCCAGGCGTCCCAT 4500
GGAAAATTTGGGTACTGCGCATGTCTTGCATAGGATTGGCGGCCTTCACACCATGGGCCATCATCCCAGCGGCTT
TTGGATATTGGCTGACTTTGAAAAC 4600
CACAAAGAGAGGAGGCGTTTTTTGGGACACACCATCACCCAAAGTCTACGCAAAAGGGGACACAACCACAGGAGT
GTACAGGATAATGGCTCGAGGGATC 4700
TTTGGCGTCTACCAAGCAGGTGTTGGAGTGATGTATGAGAACGTGTTCCACACTCTGTGGCACACGACCAGAGGA
GCTGCCATAATGAGTGGAGAAGGAA 4800

AGTTGACGCCGTACTGGGGAAGCGTTAAGGAGGACCGCATAACTTATGGGGGCCCATGGAGATTCGATCGAAAAT
GGAATGGAGTGGATGACGTGCAAAT 4900
GATTGTAGTTGAACCAGGAAAAGCAGCTGTAAATGTCCAAACAAAGCCGGGAGTGTTTCGGACTCCACACGGAGA
AATTGGAGCTGTTAGCCTGGATTAC 5000
CCTAGTGGGACATCGGGCTCACCCATCCTGGACATCAACGGTGATATCATTGGATTGTATGGGAACGGAGTTGAA
CTTGGAGATGGCTCATATGTGAGCG 5100
CCATTGTGCAGGGTGAACGGCAAGAAGAGCCCATCCCCGATGCATACAATCCAAACATGCTCAAGAAAAGGCAGC
TGACCGTGTTGGACTTGCATCCAGG 5200
ATCTGGGAAAACAAGGAAAATCTTACCCCAAATCATCAAGGATGCCATTCAACAGCGTCTCAGAACAGCTGTTCT
GGCGCCCACTCGGGTTGTTGCAGCT 5300
GAGATGGCAGAAGCTCTTAGAGGACTTCCCGTTAGATACCAAACTTCAGCGGTTCCGCGAGAACATCAGGGGAAT
GAGATAGTTGATGTCATGTGTCACG 5400
CCACCTTAACGCATAAGCTGATGTCACCAAATCGCGTCCCCAATTACAACTTGTTTGTCATGGATGAGGCCCATT
TCACAGACCCAGCCAGCATCGCCGC 5500
TAGAGGATACATATCCACCAGAGTGGAATTGGGAGAAGCCGCGGCTATCTTCATGACTGCCACTCCACCAGGAAC
GACTGACCCCTTCCCTGACTCCAAC 5600
GCTCCCATTCATGATTTGCAGGACGAGATCCCTGACAGAGCATGGAGCAGTGGGTATGAATGGATAACTGAGTAC
TCTGGGAAGACAGTATGGTTTGTGG 5700
CGAGTGTGAAAATGGGCAACGAAATCGCAGTGTGTCTACAGAGAGCCGGAAAGAGAGTCATCCAGTTAAATCGGA
AATCTTATGACACCGAGTACCCCAA 5800
ATGTAAGAATGGGGATTGGGATTTTGTCATCACCACGGACATTTCTGAAATGGGGGCCAACTTTGGAGCGAGCAG
AGTGATTGATTGTAGGAAGAGTGTG 5900
AAACCCACCATTCTGGAAGAAGGAGAAGGAAGAGTCATTCTCAGCAACCCATCGCCTATCACCAGTGCGAGTGCA
GCCCAGCGGAGAGGCAGAGTGGGCA 6000
GAAATCCAAATCAGGTTGGAGATGAGTACCATTACGGAGGGGTCACAAGTGAAGATGACACCAACCTAGCACACT
GGACAGAAGCCAAGATCATGTTGGA 6100
CAACATCCACCTGCCAAATGGGTTGGTAGCTCAGCTCTATGGACCTGAAAGGGAAAAGGCCTTCACAATGGACGG
TGAGTATCGATTGAGGGGTGAAGAA 6200
AAGAAGAACTTTCTGGAGTTAATCAGAACAGCCGACCTCCCCGTATGGCTAGCTTACAAAGTGGCTTCAAATGGA
ATACAGTACACCGATAGGAGATGGT 6300

GTTTTGATGGACCTCGGACGAATGCTATCTTAGAAGACAGCACTGAAGTAGAGATAATCACCAGAACGGGAGAAA
GGAAAATTCTAAAACCAAGATGGCT 6400
GGACGCACGGGTGTACGCAGATCACCAGGCTTTGAAGTGGTTTAAGGACTTCGCAGCAGGAAAGAGATCAGCTGT
CAGTTTTCTAGAGGTGCTTGGGCGC 6500
ATGCCAGAGCACTTCATGGGGAAAACGCGTGAAGCTCTTGATACAATGTACCTGGTTGCCACGGCAGAAAAAGGG
GGAAAAGCTCACCGAATGGCCCTGG 6600
AGGAACTGCCAGATGCACTGGAGACGGTGACACTCATCGCAGCGCTTGCCGTGATGACAGGTGGGTTCTTTCTAC
TCATGATGCAACGAAAGGGCATAGG 6700
AAAAATGGGCCTCGGGGCTCTCGTGCTCACCCTGGCCACTTTCTTCTTGTGGATGGCAGAGGTCTCAGGGACGAA
AATAGCCGGGACCTTACTCATAGCG 6800
TTGCTGCTTATGGTGGTACTCATCCCGGAGCCGGAGAAGCAAAGATCCCAAACGGACAACCAGTTGGCCGTGTTT
CTGATCTGCGTCCTCACCGTAGTGG 6900
GAATTGTGGCTGCTAATGAGTATGGCATGCTCGAAAAGACCAAGGAAGACATAAGGAATGTGTTTGGTAACAAGG
TTCAGACATCCAATGCACCTGGAAG 7000
CCTATCAAGTCTGGCGCTCGATCTACGACCAGCAACGGCTTGGGCCTTATATGGAGGTAGTACAGTGATTTTAAC
TCCACTGCTGAAACATTTGATCACC 7100
TCTGAGTATGTGACAACATCACTAGCTTCAATCAACTCACAGGCCGGCTCACTTTTTGTTTTGCCAAAAGGCATG
CCTTTCACGGATTTGGATCTGACGG 7200
TTGGACTCGTCTTTCTGGGTTGTTGGGGGCAAATCACTCTCACCACTTTTCTGACAGCTGGAGTGTTAGTAGTTC
TGCATTACGGCTATATGCTCCCTGG 7300
CTGGCAAGCTGAAGCCCTGAGGGCAGCTCAGAGACGAACAGCTGCGGGTATCATGAAGAACGCCGTTGTGGATGG
GATGGTTGCCACTGATGTGCCTGAA 7400
TTGGAAAGAACAACACCCCTAATGCAGAAGAAGGTGGGGCAAGTGTTGCTAATAGGAGTCAGTATAGCAGCTTTT
CTTGTCAATCCCAATGTCACCACCG 7500
TGCGAGAAGCCGGTGTGCTGGTGACCGCTGCTACGCTCACCCTATGGGATAATGGAGCAAGTGCCGTCTGGAATT
CAACTACAGCCACAGGACTCTGCCA 7600
CGTCATGCGAGGCAGTTACTTGGCTGGTGGTTCAATAGCTTGGACTCTCATCAAGAATGTTGATAAACCATCCTT
GAAAAGAGGAAGGCCTGGAGGAAGA 7700
ACGCTGGGTGAGCAATGGAAAGAAAAATTGAACGCCATGAACAAAGAAGAGTTTTTCAGGTACAGGAAAGAAGCC
ATAGTTGAGGTGGACCGCACAGAGG 7800

CACGCAGGGCTAGACGAGAGAATAACAAAGTGGGAGGTCATCCCGTGTCACGAGGTTCAGCAAAGCTCCGATGGA
TGGTGGAGAAAGGGTTTGTCTCCCC 7900
TGTTGGAAAGGTTGTGGACCTTGGCTGTGGGCGGGGAGGATGGTGCTATTATGCTGCCACTCTGAAAAAAGTGCA
AGAAGTTAAAGGTTATACAAAAGGA 8000
GGGGCCGGACATGAGGAACCGATGCTGATGCAAAGCTATGGTTGGAATCTGGTCACGATGAAGAGTGGAGTGGAT
GTGTTCTACAGACCTTCAGAGCCTA 8100
GTGACACCCTGCTCTGTGACATAGGGGAGTCTTCTCCAAGTCCAGACGTCGAGGAGCAACGCACTCTGCGGGTTC
TGGAAATGGCATCAGAGTGGCTACA 8200
CCGAGGACCCAGAGAATTCTGCATTAAAGTTTTGTGTCCATACATGCCAAAGGTGATAGAAAAGATGGAAACGCT
ACAACGTCGCTTTGGAGGCGGACTG 8300
GTGCGCGTTCCCCTGTCACGCAACTCAAATCACGAAATGTACTGGGTTAGTGGGGCTGCTGGGAACGTGGTACAT
GCTGTAAACATGACCAGTCAAGTTT 8400
TGCTGGGGCGAATGGACCGACCAGTCTGGAGAGGACCCAAATATGAAGAAGATGTCAACTTGGGAAGCGGGACCA
GAGCTGTAGGGAAAGGTGAGGTTCA 8500
CAGTGACCAAGGAAAAATCAAGAAGCGGATAGAGAAGCTGAAAGAAGAGTATGCAGCGACGTGGCACGAGGACCC
TGAACATCCATACCGCACTTGGACA 8600
TACCATGGAAGCTATGAAGTGAAGGCCACCGGTTCAGCCAGCTCCCTTGTCAACGGAGTGGTTAAGCTCATGAGT
AAACCTTGGGATGCCATCACTAGTG 8700
TCACCACCATGGCCATGACTGACACTACTCCCTTTGGTCAGCAGAGAGTCTTCAAAGAAAAAGTTGACACCAAGG
CGCCTGAGCCACCTGCAGGAGTTCG 8800
GGAAGTGCTGGACGAAACTACCAACTGGCTGTGGGCCTACTTGTCAAGAGAGAAAAGACCTCGCTTGTGTACGAG
AGAGGAGTTCATCCGGAAAGTCAAC 8900
AGCAACGCAGCTCTTGGAGCCATGTTTGCCGAGCAAAATCAGTGGAGCTCAGCCAGGGAGGCTGTTAGTGACCCG
GCCTTCTGGAACATGGTCGACATTG 9000
AAAGAGAGAACCACCTACGAGGGGAGTGCCACACCTGCATCTACAACATGATGGGAAAAAGAGAAAAGAAACCTG
GTGAGTTTGGGAAGGCTAAAGGAAG 9100
CAGAGCTATCTGGTTCATGTGGCTCGGAGCCCGCTACCTGGAATTCGAGGCACTCGGGTTCCTGAACGAGGACCA
TTGGCTGAGCAGGGAGAACTCTGGA 9200
GGAGGAGTGGAAGGCTCAGGCATACAGAAGCTAGGGTACATTTTGCGAGACATCTCAACGAAATCTGGAGGGAAA
ATGTATGCTGATGACACCGCAGGCT 9300

GGGACACTAGGATCACAAGGGTTGATCTGGACAATGAGGCAAAGGTGCTGGAACTTCTGGATGGGGAGCACAGGA
TGTTGGCCCGTGCTATTATAGAATT 9400
GACTTACAAACACAAAGTTGTCAAAGTAATGAGGCCAGCAGTAGGTGGAAAGACCGTGATGGATGTGATCTCTAG
AGAAGATCAAAGAGGGAGTGGGCAA 9500
GTGGTCACATATGCTCTCAACACCTTCACAAACATAGCTGTCCAGCTAGTGAGGTTGATGGAGGCTGAAGGGGTT
GTCGGTCCACAGGATGTGGAGCAGC 9600
TCCCCAGGAAAACCAAATTCGCAGTCAGGACATGGCTTTTTGAAAATGGAGAAGAGAGAGTCACCAGAATGGCAG
TGAGTGGGGATGACTGCGTTGTCAA 9700
ACCTTTGGATGACAGATTCGCGCATGCTTTACACTTCTTGAATGCGATGTCAAAGGTGAGGAAAGACATACAAGA
ATGGAAGCCATCTCAAGGCTGGCAC 9800
GACTGGCAGCAAGTCCCTTTCTGCTCGAACCACTTTCAGGAGATTGTGATGAAGGATGGTAGAAGCCTTGTTGTG
CCCtGCCGGGGACAGGATGAGTTAA 9900
TAGGCAGGGCCCGGATTTCACCAGGAGCAGGATGGAATGTGAGAGACACAGCCTGCCTAGCTAAGGCCTACGCCC
AAATGTGGCTCCTCCTCTACTTTCA 10000
CCGGAGAGACTTGCGCCTTATGGCCAACGCGATCTGCTCAGCCGTTCCGGTAGACTGGGTACCCACAGGTAGGAC
ATCATGGTCGATACACTCAAAAGGA 10100
GAGTGGATGACAACAGAAGACATGCTGCAGGTGTGGAACAGGGTATGGATTGAGGAGAATGAATGGATGAGAGAC
AAAACCCCCGTCGCCAGTTGGACCG 10200
ACGTCCCATACGTCGGAAAGAGGGAAGACATCTGGTGCGGCAGCTTGATCGGAACGCGGACAAGGGCCACCTGGG
CAGAAAACATCTATGCAGCAATAAA 10300
CCAAGTGAGAGCAATAATTGGAAATGAAAAGTATGTGGACTACATGACATCACTTAGGAGGTATGAAGACACTTT
GGTCCAGGAAGATAGAGTAATTTAA 10400
AAAACTTTTGGTAATGAGTGTAAATAGTAGTATTTATTGTAAATAGTGTAAATAAACAAATTTAAATAGGAAGTC
AGGCCGACGTAAGTCGCCACCGGAT 10500
GCTGAGTAGACGGTGCTGCCTGCGCCTCAGCCCCAGGAGGACTGGGTTAACAAATCTGACAACCGAAGGTAGGAA
AGCCCTCAAAACCGTCTCGGAAGAA 10600
GGTCCCTGCTTACTGGAGGTTGGAAGACCGTGTCAGGCCACGCAAGTGCCACTTCGCTGAGGAGTGCAGCCTGTA
CAGCCCCGGGAGGACCGGGTAAACA 10700
AAGCCGATGAGGCCCCCACGGCCCAAACCTCATCCAGGATGCAATGGATGAGGCGTAAGGACTAGAGGTTAGAGG
AGACCCCGTGGAAAAGAAAATGCGG 10800

CCCAAACTCTTTcGAAGCTGTAGAAGGAGTGGAAGGACTAGAGGTTAGAGGAGACCCCGCATTTGCATCAAAACA
GCATATTGACACCTGGGATTAGACT 10900
AGGAGATCTTCTGATCTATCTCAACATCAGCTACAAGGCACAGAGCGCCGAAATATGTAGCTGGTGGTGGGGAAG
AACACAGGATCT 10987

### <Example 4> Confirmation of K15P38 and K15P38-KNIH immunogenicity

### A. Immunogenicity analysis using ELISA assay

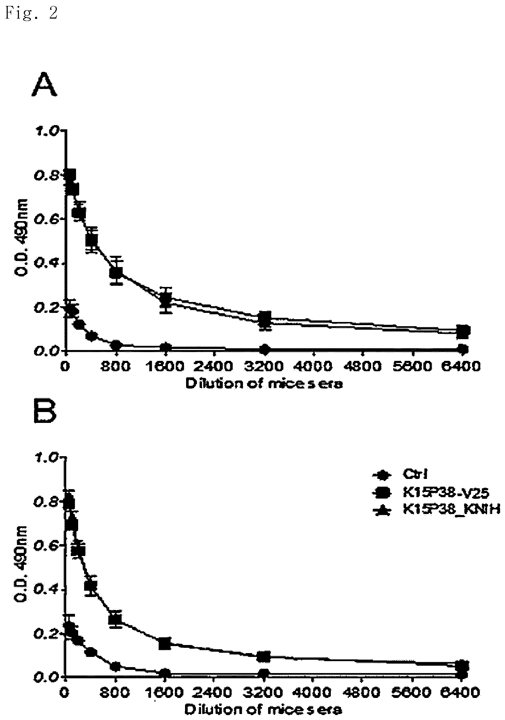

- Serum was obtained after immunizing mice with K15P38 and K15P38-KNIH viruses, respectively. Using serum obtained after immunization with each virus, an ELISA method was performed to confirm immunoglobulin G (IgG) production and cross-reaction immunogenicity of the serum for each virus. After attaching (coating) the original Japanese encephalitis virus (K15P38) or K15P38-KNIH virus to the ELISA plate before cell passage, as a result of comparing the production of virus-specific immunoglobulin G (IgG) in immune sera of each cell-passage virus (K15P38-V25) or mouse brain-passage virus (K15P38-KNIH), it was confirmed that there was no difference in the immunogenicity of cell-passage and brain-passage viruses. (Fig. 2).
- There was no difference in the antibody response to the original isolate between the two subculture methods (Fig. 2A), the immunogenicity of the brain-passage viruses was also confirmed to be similar without significant differences (Fig. 2B).

### B. Neutralizing antibody formation analysis using PRNT (Plaque Reduction Neutralization Test) analysis

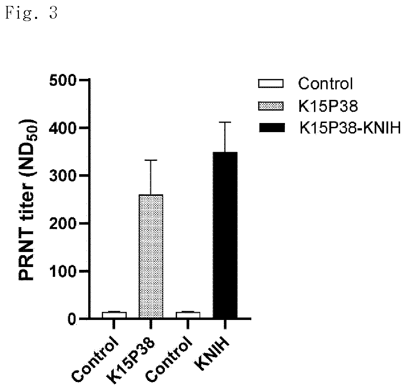

- Neutralizing antibody titers (ND₅₀, Karber formula) against the original virus (K15P38) and high titer virus (K15P38-KNIH) were compared using mouse sera immunized with Japanese encephalitis virus K15P38-KNIH.
- It was confirmed that mouse serum obtained after immunization with the K15P38-KNIH virus strain had an effective neutralizing ability against the original virus to a level similar to that of the high titer (Fig. 3).
- Neutralizing antibodies produced after immunization with high titer (K15P38-KNIH) were confirmed to have effective neutralizing ability against the original isolate and high titer, and it was confirmed that some genetic mutations did not have a significant effect on immunogenicity.

### C. Confirmation of immunogenicity of vaccine candidate using animal model (BALB/C)

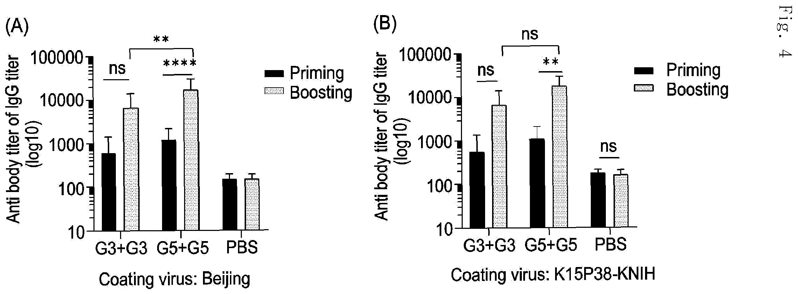

- Inactivated antigen of the vaccine strain genotype 3 (Beijing) and vaccine candidate type 5 (K15P38-KNIH) used in the Japanese encephalitis virus immunogenicity experiment was secured.
- In the group vaccinated with each antigen (8 µg/dose) of Japanese encephalitis virus genotypes 3 and 5, an increase in immunoglobulin antibody titer (total IgG) was confirmed when the same antigen was inoculated twice, and there was no significant difference in the immunogenicity of the inactivated antigen (Fig. 4).
- As a result of confirming neutralizing antibody titer (ND₅₀, Karber formula) using mouse serum induced by genotype 5 immune antigen, compared to the genotype 5 virus, a significantly lower neutralization ability was confirmed for the genotype 3 virus, which predicts that the cross-protection ability for each genotype may be low.

As above, specific parts of the present invention have been described in detail, but for those skilled in the art, it will be clear that these specific techniques are merely preferred embodiments and do not limit the scope of the present invention. Accordingly, the actual scope of the present invention will be defined by the appended claims and their equivalents.

### Accession number

Name of depository organization: Korean Collection for Type Cultures
Deposit number: KCTC150277BP
Accession date: 20220708

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT THE CASE OF AN ORIGINAL DEPOSIT

issued pursuant to Rule 7.1

TO:SHIM, Sang-mu
National Institute of Health
187 Osongsaengmyeong 2-ro, Osong-eup, Heungdeok-gu, Cheongju-si, Chungcheongbuk-do
Republic of Korea

1.INDETIFICATION OF THE MICROORGANISM
Identification reference given by the
DEPOSITOR:

Japanese Encephalitis virus (K15P38-KNIH) | Accession number given by the
INTERNATIONAL DEPOSITARY
AUTHORITY:
KCTC 150277BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTACE
This International Depositary Authority accepts the microorganism identified under I
above, which was received by it on July 8, 2022.
IV. RECEIPT OF REQUEST FOR CONVERSION
The microorganism identified under I above was received by this International Depositary
Authority on and a request to convert the original deposit to a deposit under the Budapest
Treaty was received by it on

V. INTERNATIONAL DEPOSITARY AUTHORITY

Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having
the power to represent the
International Depositary Authority
or of authorized official(s):

KIM, Son-Gun, Director
Date: July 8 2022
Form BP/4(KCTC Form 17)

## Claims

1. A high titer Japanese encephalitis virus genotype 5 K15P38-KNIH.

2. The high titer Japanese encephalitis virus genotype 5 K15P38-KNIH according to claim 1, wherein the Japanese encephalitis virus comprises the gene of SEQ ID NO: 1.

3. The high titer Japanese encephalitis virus genotype 5 K15P38-KNIH according to claim 1, wherein the Japanese encephalitis virus was produced by inoculating the mouse cerebrum.

4. The high titer Japanese encephalitis virus genotype 5 K15P38-KNIH according to claim 1, wherein the high titer may be 1×10⁸ pfu/mL or more.

5. A method for producing high titer Japanese encephalitis virus genotype 5 K15P38-KNIH, comprising:
The step of inoculating the mouse cerebrum with the isolated Japanese encephalitis virus; and
The step of obtaining Japanese encephalitis virus from mouse cerebrum.

6. The method for producing high titer Japanese encephalitis virus genotype 5 K15P38-KNIH according to 5, wherein the isolated Japanese encephalitis virus is Japanese encephalitis virus genotype 5.

7. The method for producing high titer Japanese encephalitis virus genotype 5 K15P38-KNIH according to 5, further comprising a step of subculture in Vero cells.

8. The method for producing high titer Japanese encephalitis virus genotype 5 K15P38-KNIH according to 5, wherein the inoculation may be done twice or more.

9. A vaccine composition comprising the Japanese encephalitis virus of any one of claims 1 to 4.

10. The vaccine composition according to claim 9, further comprising a pharmaceutically acceptable carrier or excipient.

11. A vaccination method against Japanese encephalitis comprising a step of administering the vaccine composition of claim 9 or 10 to a non-human animal.
